# EUROPEAN PATENT APPLICATION

(11) **EP 4 442 772 A1**
(43) Date of publication of application: **09.10.2024**
(21) Application number: 24168682.3
(22) Date of filing: 05.04.2024
(51) Int. Cl.: C09D 5/14

(54) **ANTIMICROBIAL CUPROUS OXIDE-CONTAINING COATINGS**

(30) Priority: 06.04.2023 US 202363457602 P
(71) Applicant: Vapor Technologies, Inc., Longmont CO 80503 (US)
(72) Inventor: SULLIVAN, Patrick Anthony, Longmont, 80503 (US); ANTON, Bryce Randolph, Longmont, 80503 (US)
(74) Representative: Keltie LLP

(57) **Abstract**

A coated article having an antimicrobial coating is provided. The coated article includes a substrate and at least one adhesion layer disposed over the substrate. The at least one adhesion layer includes a component selected from the group consisting of a copper oxide, a zirconium oxide, and combinations thereof. Advantageously, an antimicrobial copper suboxide layer disposed over the at least one adhesion layer.

## Description

### CROSS-REFERENCE TO RELATED APPLICATIONS

This application claims the benefit of U.S. provisional application Serial No. 63/457,602 filed April 6, 2023, the disclosure of which is hereby incorporated in its(their) entirety by reference herein.

### TECHNICAL FIELD

In at least one aspect, the present invention is related to antimicrobial coatings and to articles coated with such coatings.

### BACKGROUND

Coatings with bioactive properties can be useful for various purposes. Bioactive refers to a material that has biological or physiological effects on living things. For example, a bioactive material includes a material resulting in a modification in the normal biological function or a physiological mechanism of a living thing. Bioactive, as used herein, includes beneficial and detrimental effects on microorganisms or changes to the normal functioning of a microorganism. One common bioactive material is antimicrobial substances. Antimicrobial coatings serve many purposes. Generally, antimicrobial coatings inhibit the growth or kill microbes like viral, bacterial, or fungal organisms. One particularly relevant example includes preventing the spread of communicable diseases by using antimicrobial materials. Antimicrobial materials may also serve hygienic purposes. The desire to prevent the spread of disease and for heightened hygiene has resulted in significant efforts to develop antimicrobial materials. The use of antimicrobial materials in health care facilities and health treatments can provide significant benefits. Health facilities, such as hospitals, present unique environments that combine high concentrations of germs and individuals with vulnerable immunities. Therefore, facilities such as hospitals benefit from antimicrobial surfaces. Antimicrobial materials for medical equipment can reduce the burden of disinfecting and prevent the spread of disease. Further, affordable antimicrobial surfaces could present benefits on any surface that comes into contact with living things. For example, surfaces involved in cooking or commonly touched surfaces like doorknobs could benefit from antimicrobial properties. Even primarily decorative surfaces, if affordable, could benefit from antimicrobial characteristics and assist in inhibiting the spread and growth of harmful or undesirable antimicrobial life.

Producing antimicrobial materials can be difficult and expensive. Further, antimicrobial properties may have other undesirable properties. For example, some antimicrobial materials may be too soft. Other antimicrobial materials may have poor abrasion resistance. Microban^{®} sells antimicrobial additives like silver phosphate glass that can be mixed into paints. In some applications, the appearance of paint may be undesirable. Some antimicrobial coatings may use nanoparticle vapor deposition to deposit nanoparticles with antimicrobial properties on the surface of a coating. For example, ABACO^{®} from Protec, is an antimicrobial coating using nanoparticles. However, the use of nanoparticles can be complex and expensive. Further such coatings may have delicate surfaces or poor abrasion resistance. Another example of antimicrobial materials includes various metals. For example, silver is known to have antimicrobial properties. However, silver can be expensive and its properties may not be suitable for many applications. For example, the appearance or abrasion resistance of silver may be unsuitable for certain applications.

Accordingly, there is a need for an antimicrobial coating that solves one or more of these problems or offers an alternative to current antimicrobial materials.

### SUMMARY

In at least one aspect, a coated article having an antimicrobial coating is provided. The coated article includes a substrate and at least one adhesion layer disposed over the substrate. The at least one adhesion layer includes a component selected from the group consisting of a copper oxide, a zirconium oxide, and combinations thereof. Advantageously, an antimicrobial copper suboxide layer is disposed over the at least one adhesion layer. Characteristically, the antimicrobial copper suboxide layer is at least 5 times thicker than the at least one adhesion layer, even if the antimicrobial copper suboxide is a multilayer structure.

In another aspect, a coated article having an antimicrobial coating is provided. The coated article includes a substrate and a copper oxide suboxide adhesion layer disposed over the substrate. An antimicrobial copper suboxide layer is disposed over the at least one adhesion layer. Characteristically, the antimicrobial copper suboxide layer is at least 5 times thicker than the at least one adhesion layer, even if the antimicrobial copper suboxide is a multilayer structure.

In another aspect, a coated article having an antimicrobial coating is provided. The coated article includes a substrate and a cupric oxide adhesion layer disposed over the substrate. A copper suboxide layer is disposed over and optionally contacts the cupric oxide adhesion layer. Advantageously, the coatings on the coated article do not include metals other than copper.

The foregoing summary is illustrative only and is not intended to be in any way limiting. In addition to the illustrative aspects, embodiments, and features described above, further aspects, embodiments, and features will become apparent by reference to the drawings and the following detailed description.

### BRIEF DESCRIPTION OF THE DRAWINGS

For a further understanding of the nature, objects, and advantages of the present disclosure, reference should be had to the following detailed description, read in conjunction with the following drawings, wherein like reference numerals denote like elements and wherein:
FIGURE 1A. Cross section of a coated article having an antimicrobial layer.
FIGURE 1B. Cross section of a coated article having an antimicrobial layer with a transparent oxide overcoat.
FIGURE 2. EDS scans of a substrate coated with an adhesion layer and a copper suboxide layer.
FIGURES 3A and 3B. SEM micrographs of a copper suboxide layer.
FIGURE 4. X-ray diffraction patterns of a copper suboxide layer aligned with copper and copper oxide (Cu₂O) peaks.

### DETAILED DESCRIPTION

Reference will now be made in detail to presently preferred compositions, embodiments and methods of the present invention, which constitute the best modes of practicing the invention presently known to the inventors. The Figures are not necessarily to scale. However, it is to be understood that the disclosed embodiments are merely exemplary of the invention that may be embodied in various and alternative forms. Therefore, specific details disclosed herein are not to be interpreted as limiting, but merely as a representative basis for any aspect of the invention and/or as a representative basis for teaching one skilled in the art to variously employ the present invention.

Except in the examples, or where otherwise expressly indicated, all numerical quantities in this description indicating amounts of material or conditions of reaction and/or use are to be understood as modified by the word "about" in describing the broadest scope of the invention. Practice within the numerical limits stated is generally preferred. Also, unless expressly stated to the contrary: percent, "parts of," and ratio values are by weight; molecular weights provided for any polymers refers to weight average molecular weight unless otherwise indicated; the description of a group or class of materials as suitable or preferred for a given purpose in connection with the invention implies that mixtures of any two or more of the members of the group or class are equally suitable or preferred; description of constituents in chemical terms refers to the constituents at the time of addition to any combination specified in the description, and does not necessarily preclude chemical interactions among the constituents of a mixture once mixed; the first definition of an acronym or other abbreviation applies to all subsequent uses herein of the same abbreviation and applies mutatis mutandis to normal grammatical variations of the initially defined abbreviation; and, unless expressly stated to the contrary, measurement of a property is determined by the same technique as previously or later referenced for the same property.

It is also to be understood that this invention is not limited to the specific embodiments and methods described below, as specific components and/or conditions may, of course, vary. Furthermore, the terminology used herein is used only for the purpose of describing particular embodiments of the present invention and is not intended to be limiting in any way.

It must also be noted that, as used in the specification and the appended claims, the singular form "a," "an," and "the" comprise plural referents unless the context clearly indicates otherwise. For example, reference to a component in the singular is intended to comprise a plurality of components.

The term "comprising" is synonymous with "including," "having," "containing," or "characterized by." These terms are inclusive and open-ended and do not exclude additional, unrecited elements or method steps.

The phrase "consisting of" excludes any element, step, or ingredient not specified in the claim. When this phrase appears in a clause of the body of a claim, rather than immediately following the preamble, it limits only the element set forth in that clause; other elements are not excluded from the claim as a whole.

The phrase "consisting essentially of" limits the scope of a claim to the specified materials or steps, plus those that do not materially affect the basic and novel characteristic(s) of the claimed subject matter.

With respect to the terms "comprising," "consisting of," and "consisting essentially of," where one of these three terms is used herein, the presently disclosed and claimed subject matter can include the use of either of the other two terms.

It should also be appreciated that integer ranges explicitly include all intervening integers. For example, the integer range 1-10 explicitly includes 1, 2, 3, 4, 5, 6, 7, 8, 9, and 10. Similarly, the range 1 to 100 includes 1, 2, 3, 4.... 97, 98, 99, 100. Similarly, when any range is called for, intervening numbers that are increments of the difference between the upper limit and the lower limit divided by 10 can be taken as alternative upper or lower limits. For example, if the range is 1.1. to 2.1 the following numbers 1.2, 1.3, 1.4, 1.5, 1.6, 1.7, 1.8, 1.9, and 2.0 can be selected as lower or upper limits. In the specific examples set forth herein, concentrations, temperature, and reaction conditions (e.g. pressure, pH, etc.) can be practiced with plus or minus 50 percent of the values indicated rounded to three significant figures. In a refinement, concentrations, temperature, and reaction conditions (e.g., pressure, pH, etc.) can be practiced with plus or minus 30 percent of the values indicated rounded to three significant figures of the value provided in the examples. In another refinement, concentrations, temperature, and reaction conditions (e.g., pH, etc.) can be practiced with plus or minus 10 percent of the values indicated rounded to three significant figures of the value provided in the examples.

The term "copper suboxide" means a copper oxide having a higher copper to oxygen atom ratio greater than the copper to oxygen ratio in cupric oxide (CuO). Therefore, the copper to oxygen ratio is greater than 1:1. For example, cuprous oxide (Cu₂O) is considered a copper suboxide.

The term "a copper oxide" means a compound having formula CuₓO where x is from 2.2 to 0.6. In a refinement, z is at least 0.6, 0.7, 0.9, or 0.95 and at most 2.2, 2.0, 1.8, 1.6, 1.4 or 1.2.

The term "a zirconium oxide" means a compound having formula Zr_{y}O₂ where y is from 3 to 0.6.

### Abbreviations:

"EDS" means Energy-dispersive X-ray spectroscopy.
"SEM" means scanning electron microscopy.

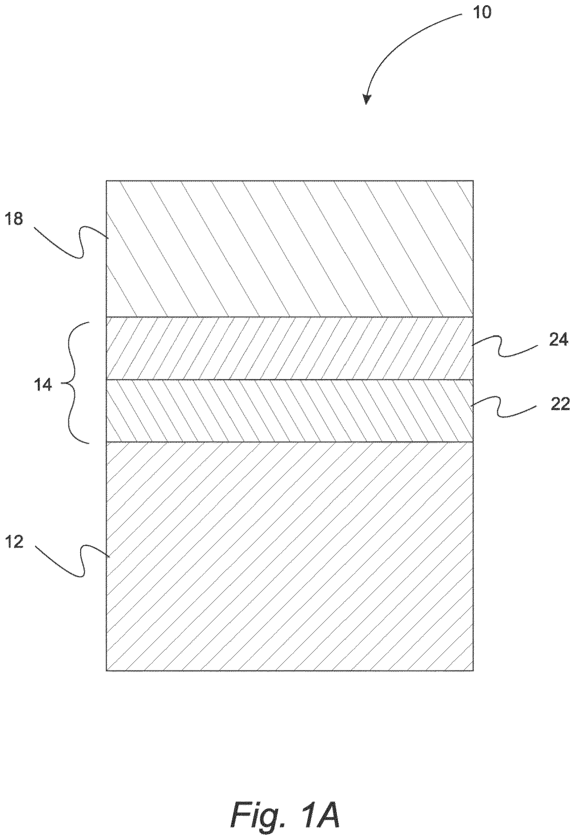

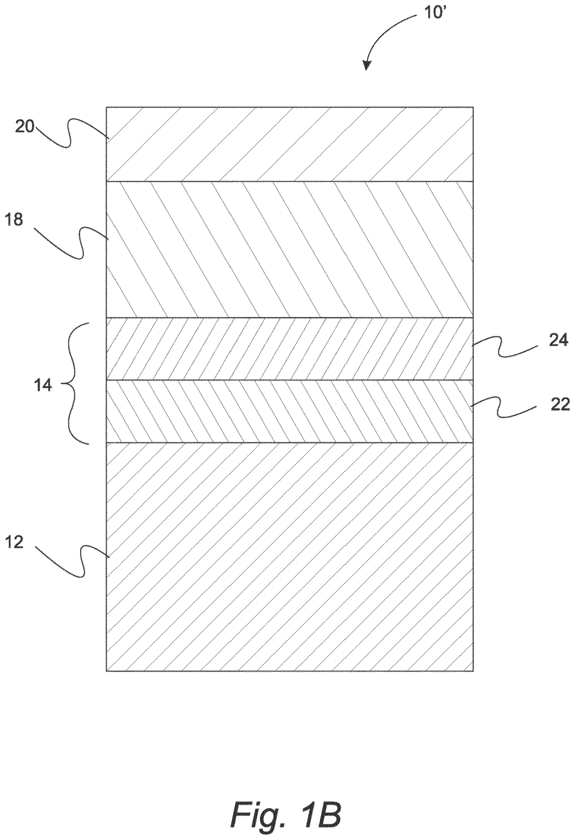

Referring to Figures 1A and 1B, schematics of a coated article having an antimicrobial coating are provided. Coated article 10 includes substrate 12 and at least one adhesion layer 14 disposed over the substrate. The at least one adhesion layer 14 includes a component selected from the group consisting of a copper oxide, a zirconium oxide, and combinations thereof. Antimicrobial copper suboxide layer 18 is disposed over the at least one adhesion layer 14. Characteristically, the antimicrobial copper suboxide layer 18 is at least 5 times thicker than the at least one adhesion layer 14. In a refinement, the antimicrobial copper suboxide layer 18 has a thickness greater than 0.5 microns. In a further refinement, the copper suboxide layer 18 has a thickness from about 0.5 microns to about 10 microns. Advantageously, the at least one adhesion layer 14 and the copper suboxide layer 18 can be made by chemical vapor deposition or by physical vapor depositions. Suitable physical vapor deposition techniques include arc deposition and reactive sputtering. Typically, the at least one adhesion layer 14 has a thickness from about 20 nm to 200 nm. Figure 1B provides a variation in which the copper suboxide layer 18 is coated with a top layer 20.

In another aspect, top layer 20 is a transparent metal oxide layer 20 such as a layer including a zirconium oxide.

In another aspect, top layer 20 is an antimicrobial silane-containing layer as disclosed in US Pat. Pub. No. 2022/0235232; the entire disclosure of which is hereby incorporated by reference. In this regard, the antimicrobial silane-containing layer can include a microbial inhibition material having a first silane compound and a metal ion wherein the first silane compound includes a functional group bound to the metal ion. For example, the antimicrobial silane-containing layer can include an antimicrobial material. In particular, the antimicrobial silane-containing layer includes a first silane compound and a metal ion. Characteristically, the silane compound includes a functional group bound to the metal ion. In a further refinement, the antimicrobial silane-containing layer further includes a fingerprint inhibition material, which comprises a silane compound (e.g., a second silane compound) that may be preferably hydrophobic.

In another aspect, the first silane compound is described by Formula (I): 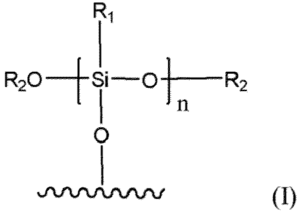 wherein:
R₁ is selected from the group consisting of -C₁-Cₓ₊₁ alkyl-S(R₃)_{y}(M), -C₂-Cₓ₊₂ alkenyl-S(R₃)_{y}(M), -C₃-Cₓ₊₃ alkynyl-S(R₃)_{y}(M), -C₁₋Cₓ alkyl-N(R₃)_{y}(M), -C₂-Cₓ alkenyl-N(R³)_{y}(M), -C₃-Cₓ alkynyl-N(R₃)_{y}(M), -C₁-Cₓ₊₁ alkyl-P(R₃)_{y}(M), -C₂-Cₓ₊₂ alkenyl-P(R₃)_{y}(M), and -C₃-Cₓ₊₃ alkynyl-P(R₃)_{y}(M);
each R₂ is the same or different and is a bond to another Si atom of a repeating structure, or is selected from the group consisting of hydrogen, -C₁-C₁₀ alkyl, -C₂-C₁₀ alkenyl, -C₃-C₁₀ alkynyl;
n is a positive integer (e.g., from 1 to 500 or greater);
x is a positive integer (e.g., from 1 to 20 or greater);
y is a positive integer (e.g., from 1 to 4 or greater); and
M is a metal ion or metal.

In another aspect, n is a positive integer greater than or equal to 1, 2, 10, 20, 50, 100, 200, 300, 400, or 450. In a refinement, n is a positive integer less than or equal to 500, 450, 400, 300, 200, 100, 50, 20, 10, 5, or 2. Combinations of the above-referenced ranges are also contemplated.

In another aspect, x is a positive integer greater than or equal to 1, 2, 4, 6, 8, 10, 12, 14, 16, or 18. In a refinement, x is a positive integer less than or equal to 20, 18, 16, 14, 12, less than or equal to 10, 8, 6, 4, or 2. Combinations of the above-referenced ranges are also contemplated.

In another aspect, y is a positive integer greater than or equal to 1, 2, 3. In a refinement, y is less than or equal to 4, 3, or 2. Combinations of the above-referenced ranges are also contemplated.

In a refinement, M is a metal ions selected from the group consisting of copper ions (e.g., Cu⁺, Cu²⁺), titanium ions (e.g., Ti³⁺, Ti⁴⁺), vanadium ions (e.g., V²⁺, V³⁺), chromium ions (e.g., Cr²⁺, Cr3⁺), manganese ions (e.g., Mn²⁺, Mn⁴⁺), iron ions (e.g., Fe²⁺, Fe³⁺), cobalt ions (e.g., Co²⁺, Co³⁺), nickel ions (e.g., Ni²⁺), zinc ions (e.g., Zn²⁺), zirconium ions (e.g., Zr²⁺), palladium ions (e.g., Pd²⁺), cadmium ions (e.g., Cd²⁺), platinum ions (e.g., Pt²⁺, Pt⁴⁺), gold ions (e.g., Au⁺, Au³⁺), mercury ions (e.g., Hg²⁺), terbium ions (e.g., Tb³⁺), tungsten ions (e.g., W⁴⁺), aluminum ions (e.g., Al³⁺), gallium ions (e.g., Ga³⁺), germanium ions (e.g., Ge²⁺), arsenic ions (e.g., As³⁺), selenium ions (e.g., Se²⁺) tin ions (e.g., Sn²⁺), antimony ions (e.g., Sb³⁺), tellurium ions (e.g., Te²⁺), lead ions (e.g., Pb⁴⁺), bismuth ions (e.g., Bi³⁺), and/or combinations thereof. In another refinement, M is a metal selected from the group consisting of Ag, Cu, Ti, V, Cr, Mn, Fe, Co, Ni, Zn, Zr, Pd, Cd, Pt, Au, Hg, W, Al, Ga, Ge, Tb, As, Se, Sn, Sb, Te, Pb, and Bi.

In some refinements, each R₂ is the same or different and is a bond to another Si atom of a repeating structure, or is selected from the group consisting of hydrogen, -C₁-C₁₀ alkyl, -C₂-C₁₀ alkenyl, -C₃-C₁₀ alkynyl.

In a refinement, each carbon atom and/or hydrogen atom in R₁ and R₂ is independently optionally substituted with deuterium, halogen, -OH, -CN, -O R₃, -CO₂H, -C(O)O R₃, -C(O)NH₂, C(O)NH(C₁-C₁₀ alkyl), -C(O)N(C₁-C₁₀ alkyl)₂, SC₁-C₁₀ alkyl, -S(O)C₁-C₁₀ alkyl, - S(O)₂C₁-C₁₀ alkyl, -S(O)NH(C₁-C₁₀ alkyl), -S(O)₂NH(C₁-C₁₀ alkyl), -S(O)N(C₁C₁₀ alkyl)₂, - S(O)₂N(C₁-C₁₀ alkyl)₂, -N R₃, -NH₂, -C₂H₈N₂, -NH(C₁-C₁₀ alkyl), -P(C₁-C₁₀ alkyl)₂, -P(O)(C₁-C₁₀ alkyl)₂, -PO₃H₂, or -Si(-OC₁-C₁₀ alkyl)₃,

In a refinement, each R₃ is selected from the group consisting of a bond, hydrogen, deuterium, -C₁-C₁₀ alkyl, -C₂-C₁₀ alkenyl, -C₂-C₁₀ alkynyl, -C₃-C₁₀ cycloalkyl, and -C₁-C₁₀ alkyl-O-C₁-C₁₀ alkyl, optionally substituted and n is a positive integer.

In some variations, the first silane compound is immobilized on a substrate. The first silane compound can be chemically bound to the substrate by covalent bonds and/or noncovalent bonds.

The antimicrobial silane-containing layer is not limited by the amount of the microbial inhibition material. For example, the microbial inhibition material can be present in an amount from about 0.1 wt. %, to about 100 wt. %.

In a variation, the antimicrobial silane-containing layer further includes a fingerprint inhibition material. The fingerprint inhibition material includes a second silane compound. The second silane compound can provide hydrophobic properties. In a refinement, the fingerprint inhibition material includes an invisible fingerprint material that is hydrophobic and oleophilic and/or an anti-fingerprint material that is hydrophobic and oleophobic. In a variation, the second silane compound is described by structure (II): 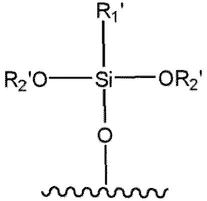 wherein:
R₁' is selected from the group consisting of -C₁-C₂₀ alkyl, -C₂-C₂₀ alkenyl, and -C₃-C₂₀ alkynyl,
any of which may be optionally at least partially halogenated (e.g., fluorinated); and
R₂' is the same or different and is a linkage to a substrate, or is selected from the group consisting of hydrogen, -C₁-C₁₀ alkyl, -C₂-C₁₀ alkenyl, and -C₃-C₁₀ alkynyl. In a refinement,
R₁' and R₁' can be the same as set forth above for Ri and R₂ respectively.

The antimicrobial silane-containing layer is not limited by the amount of fingerprint inhibition material. For example, the microbial inhibition material can be present in an amount from about 0.1 wt. %, to about 100 wt. %.

In a variation, each carbon atom and/or hydrogen atom in R₁' and R₂' is independently optionally substituted with deuterium, halogen, -OH, -CN, -O R₃, -CO₂H, -C(O)O R₃, -C(O)NH₂, C(O)NH(C₁-C₁₀ alkyl), -C(O)N(C₁-C₁₀ alkyl)₂, SC₁-C₁₀ alkyl, -S(O)C₁-C₁₀ alkyl, - S(O)₂C₁-C₁₀ alkyl, -S(O)NH(C₁-C₁₀ alkyl), -S(O)₂NH(C₁-C₁₀ alkyl), -S(O)N(C₁C₁₀ alkyl)₂, - S(O)₂N(C₁-C₁₀ alkyl)₂, -N R₃, -NH₂, -C₂H₈N₂, -NH(C₁-C₁₀ alkyl), -P(C₁-C₁₀ alkyl)₂, -P(O)(C₁-C₁₀ alkyl)₂, -PO₃H₂, or -Si(-OC₁-C₁₀ alkyl)₃; and each R₃ is selected from the group consisting of hydrogen, deuterium, -C₁-C₁₀ alkyl, -C₂-C₁₀ alkenyl, -C₂-C₁₀ alkynyl, -C₃-C₁₀ cycloalkyl, and -C₁-C₁₀ alkyl-O-C₁-C₁₀ alkyl, optionally substituted.

In one variation, the at least one adhesion layer includes a copper oxide layer composed of a copper oxide that is different than the antimicrobial copper suboxide layer. The copper oxide can be described by the formula CuₓO where x is from 2.2 to 0.6. In a refinement, x is at least 0.6, 0.7, 0.9, 0.9, or 0.95 and at most 2.2, 2.0, 1.8, 1.6, 1.4 or 1.2. In a refinement, the at least one adhesion layer is a cupric oxide (i.e., Cu(II)O) layer. In a refinement, the copper oxide layer has a thickness from about 10 to 200 nm. In a further refinement, the copper oxide layer has a thickness of at least 5 nm, 10 nm, 20 nm, 30 nm, 40 nm, or 50 nm and a thickness of at most 300 nm, 250 nm, 2000 nm, 180 nm, 150 nm, 120 nm, or 100 nm.

In another variation, the at least one adhesion layer includes a zirconium oxide layer that is composed of a zirconium oxide. The zirconium oxide can be described by the formula Zr_{y}O₂ where y is from 3 to 0.6. In a refinement, y is at least 0.6, 0.7, 0.8, 0.9, or 0.95 and at most 3, 2.5, 2.0, 1.8, 1.6, 1.4 or 1.2. In another refinement, y is about 1 and the at least one adhesion layer includes a zirconia layer (ZrO₂). In a refinement, the zirconium oxide layer has a thickness from about 10 to 300 nm. In a further refinement, the zirconium oxide layer has a thickness of at least 5 nm, 10 nm, 20 nm, 30 nm, 40 nm, 50 nm, or 80 nm and a thickness of at most 400 nm, 300 nm, 250 nm, 2000 nm, 180 nm, 150 nm, 120 nm, or 100 nm.

In another variation, the at least one adhesion layer is a bilayer that includes a zirconium oxide layer and cupric oxide layer. For example, as depicted in Figure 1A and 1B, the at least one adhesion layer 14 includes a zirconium oxide layer 22 disposed over and optionally contacting substrate 12 with a copper oxide layer 24 disposed over and optionally contacting zirconium oxide layer 20. As set forth above, the zirconium oxide can be described by the formula Zr_{y}O₂ where y is from 3 to 0.6. In a refinement, y is at least 0.6, 0.7, 0.8, 0.9, or 0.95 and at most 3, 2.5, 2.0, 1.8, 1.6, 1.4 or 1.2. In another refinement, y is about 1 and the at least one adhesion layer includes a zirconia layer (ZrO₂). In a refinement, copper oxide layer 24 is composed of Cu₂O, CuO, Cu₄O₃ or combinations thereof. In a refinement, the copper oxide layer 24 is composed of a copper oxide described by the formula CuₓO where x is from 2.2 to 0.6. In a refinement, x is at least 0.6, 0.7, 0.9, 0.9, or 0.95 and at most 2.2, 2.0, 1.8, 1.6, 1.4 or 1.2. In a refinement, the at least one adhesion layer is a cupric oxide (i.e., Cu(II)O) layer. In a refinement, the zirconium oxide layer and the copper oxide layer each independently have a thickness from about 10 to 300 nm. In a further refinement, zirconium oxide layer and the cupric oxide layer each independently have a thickness of at least 5 nm, 10 nm, 20 nm, 30 nm, 40 nm, 50 nm, or 80 nm and a thickness of at most 400 nm, 300 nm, 250 nm, 2000 nm, 180 nm, 150 nm, 120 nm, or 100 nm.

In a variation, the copper suboxide layer 18 is composed of a compound having formula Cu_{z}O where z is from 3 to 1.1. In a refinement, z is at least 1.1, 1.3, 1.5, 1.6, 1.7, or 1.8 and at most 3, 2.7, 2.5, 2.4, 2.3, 2.2, or 2.1. In a refinement, the copper suboxide layer includes cuprous oxide (Cu₂O).

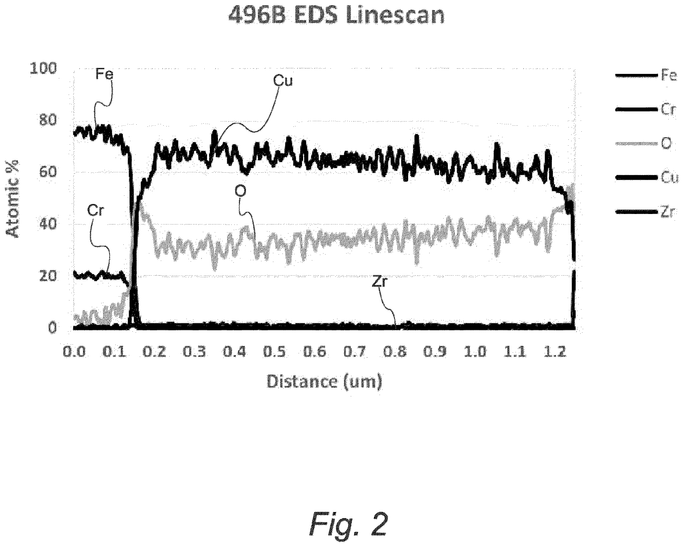

In a variation, the copper suboxide layer 18 has a uniform stoichiometry to within 20 mole % throughout the thickness of the copper suboxide layer. In a refinement, the copper suboxide layer has a uniform stoichiometry to within 10 mole % throughout the thickness of the copper suboxide layer. In another variation, the copper atom percent in the copper suboxide layer decreases at increasing distances from the substrate. In other words, the ratio of copper to oxygen decrease at increasing distances from the substrate. Figure 2 provides an EDS scan providing the elemental analysis of a stainless steel substrate. The analysis shows that in the copper suboxide layer, the ratio of copper to oxygen decrease at increasing distances from the substrate.

In a variation, the copper suboxide layer 18 has a dark gray non-iridescent color. In accessing color, copper suboxide layer 18 can be characterized by Lab color space coordinates L*, a*, and b* relative to a CIE standard illuminant such as F2, L2, D65, and D50. In a refinement, copper suboxide layer 18 has color space coordinates L* from 35 to 55, a* from -5 to 5, and b* from -10 to -4 CIE standard illuminant D65.

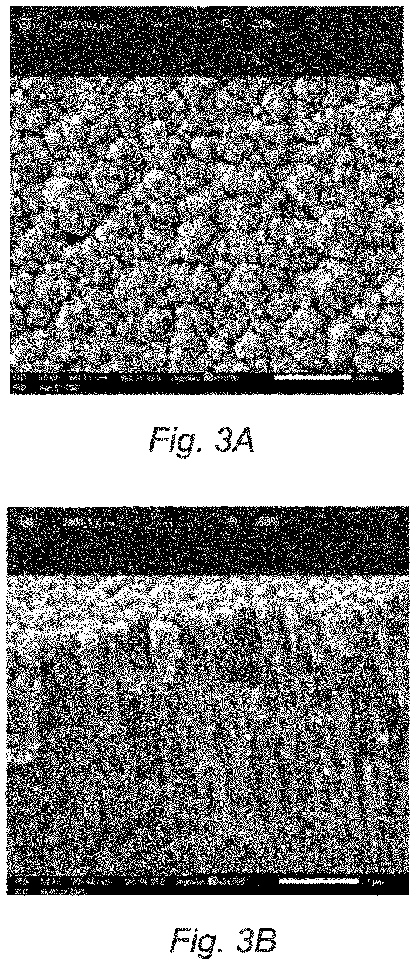

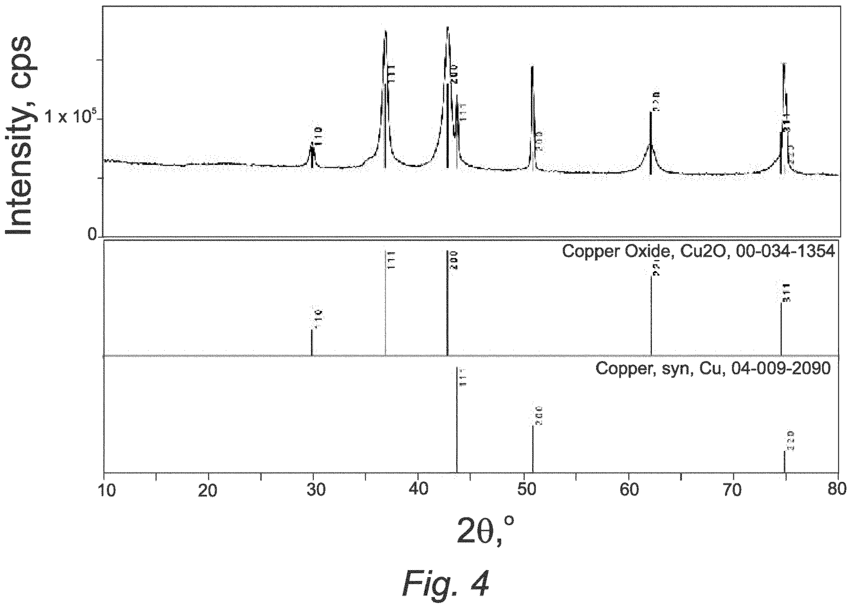

In a variation, the copper suboxide layer includes a plurality of crystalline columns extending from the substrate. In a refinement, the columns expand (i.e., the cross section of the columns increase) along a direction perpendicular to the substrate. Figures 3A and 3B provide SEM micrographs for copper suboxide layer 18 showing the columnar growth. Figure 4 provides the X-ray diffraction patterns of copper suboxide layer revealing that the crystal are not oriented.

In a variation, the adhesion layer includes a copper oxide with an antimicrobial copper suboxide layer disposed over the adhesion layer. As set forth above, the antimicrobial copper suboxide layer is at least 5 times thicker than the adhesion layer. Advantageous, the combination of the adhesion layer and the antimicrobial copper suboxide layer are free of other metals other than copper. In this context, free means that both the adhesion layer and the antimicrobial copper suboxide layer independently include less than 1 mole percent of other metals.

Table 1 provides properties of the antimicrobial copper suboxide layer (e.g., cuprous oxide). Table 2 provides properties of an adhesion layer that includes a copper oxide (e.g., a cupric oxide layer). Table 3 provides properties of an adhesion layer that includes a zirconium oxide (e.g., a zirconium oxide layer).

**Table 1. Antimicrobial copper suboxide layer properties**

| **Bulk (cuprous oxide) layer** | Max | Min | Nominal | Notes |
|---|---|---|---|---|
| color range (L*, a*, b*) | (55,5, 5) | (35, -5,-10) | (43, 0, - 4) | CIELAB, 0/10 geometry, D65 source, SPI mode |
| thickness (microns) | 10 | 0.5 | 2 | |
| grain size (microns) | 0.5 | 0.05 | 0.2 | |
| composition (at% Cu) | 80 | 50 | 67 | |
| composition (at% O) | 50 | 20 | 33 | |
| Phase | copper | cupric | cuprous | |
| Orientations | | | | (2,0,0), (1,1,1), (3,1,1) no obviously preferred orientation |

**Table 2. Cupric oxide layer properties**

| **Adhesion (cupric oxide) layer** | **Max** | **Min** | **Nominal** |
|---|---|---|---|
| thickness (microns) | 0.5 | 0.01 | 0.05 |
| composition (at% Cu) | 67 | 40 | 50 |
| composition (at% O) | 60 | 33 | 50 |

**Table 3. A zirconium-containing layer properties**

| **Adhesion (zirconia) layer [Optional]** | **Max** | **Min** | **Nominal** |
|---|---|---|---|
| thickness (microns) | 0.5 | 0.01 | 0.15 |
| composition (at% Zr) | 100 | 33 | 33 |
| composition (at% O) | 67 | 0 | 67 |

Table 4 provides the color properties for a stainless steel substrate coated

**Table 4. Color properties of a coated article**

| **Data Name** | **L*(F2)** | **a*(F2)** | **b*(F2)** | **L*(D65)** | **a*(D65)** | **b*(D65)** | **Gloss (Calc)** |
|---|---|---|---|---|---|---|---|
| Example 1 | 43.33 | 0.34 | -4.83 | 43.56 | 0.63 | -4.72 | 0.9649 |
| Example 2 | 43.33 | 0.33 | -4.84 | 43.56 | 0.63 | -4.72 | 0.9649 |
| Example 3 | 43.25 | 0.65 | -4.32 | 43.5 | 0.96 | -4.13 | 0.9658 |
| Example 4 | 43.92 | -1.04 | -5.87 | 44.47 | -1.36 | -4.83 | 0.9225 |
| Example 5 | 42.74 | 2.47 | -2.32 | 43.09 | 3.33 | -1.76 | 0.9101 |
| | | | | | | | |
| min | 42.74 | -1.04 | -5.87 | 43.09 | -1.36 | -4.83 | 0.9101 |
| max | 43.92 | 2.47 | -2.32 | 44.47 | 3.33 | -1.76 | 0.9658 |

Table 5 provides examples of a multilayer coating on a stainless steel substrate.

**Table 5. Examples of a multilayered coating**

| **Sample ID** | **Layer1** | | **Layer2** | | **Layer3** | |
|---|---|---|---|---|---|---|
| | **material** | **approx. thickness (nm)** | **material** | **approx. thickness (nm)** | **material** | **approx. thickness (nm)** |
| **Example 6** | copper oxide | 50 | copper oxide | 1200 | none | |
| **Example 7** | copper oxide | 50 | copper oxide | 1200 | | |
| **Example 8** | zirconia | 100 | copper oxide | 1200 | zirconia | 50 |

While exemplary embodiments are described above, it is not intended that these embodiments describe all possible forms of the invention. Rather, the words used in the specification are words of description rather than limitation, and it is understood that various changes may be made without departing from the spirit and scope of the invention. Additionally, the features of various implementing embodiments may be combined to form further embodiments of the invention.

Examples of the invention will now be expressed by the following numbered clauses:
1. A coated article comprising: a substrate; at least one adhesion layer disposed over the substrate, the at least one adhesion layer including a component selected from the group consisting of a copper oxide, a zirconium oxide, and combinations thereof; and an antimicrobial copper suboxide layer disposed over the at least one adhesion layer wherein the antimicrobial copper suboxide layer is at least 5 times thicker than the at least one adhesion layer.
2. The coated article of clause 1, wherein the antimicrobial copper suboxide layer includes a compound having formula Cu_{z}O where z is from 2.2 to 1.1.
3. The coated article of any of the previous clauses, wherein the antimicrobial copper suboxide layer includes cuprous oxide.
4. The coated article of any of the previous clauses, wherein the at least one adhesion layer includes a compound having formula CuₓO where x is from 2.2 to 0.6.
5. The coated article of any of the previous clauses, wherein the at least one adhesion layer includes a compound having formula Zr_{y}O₂ where y is from 3 to 0.6.
6. The coated article of any of the previous clauses, wherein the antimicrobial copper suboxide layer has a uniform stoichiometry to within 20 mole % throughout the antimicrobial copper suboxide layer.
7. The coated article of any of the previous clauses, wherein a copper atom percent in the antimicrobial copper suboxide layer decreases at increasing distances from the substrate.
8. The coated article of any of the previous clauses, wherein the antimicrobial copper suboxide layer has a thickness greater than 0.5 microns.
9. The coated article of any of the previous clauses, wherein the antimicrobial copper suboxide layer has a thickness from about 0.5 microns to about 10 microns.
10. The coated article of any of the previous clauses, wherein the antimicrobial copper suboxide layer has a gray non-iridescent color.
11. The coated article of any of the previous clauses, wherein the at least one adhesion layer has a thickness less than about 250 nm.
12. The coated article of any of the previous clauses, wherein the at least one adhesion layer has a thickness from about 10 nm to about 200 nm.
13. The coated article of any of the previous clauses, wherein the antimicrobial copper suboxide layer includes a plurality of crystalline columns extending from the substrate.
14. The coated article of any of the previous clauses, wherein the antimicrobial copper suboxide layer has color space coordinates L* from 35 to 55, a* from -5 to 5, and b* from -10 to -4 for illuminants D65.
15. The coated article of any of the previous clauses, further comprising a transparent metal oxide layer disposed over the antimicrobial copper suboxide layer.
16. The coated article of any of the previous clauses, further comprising an antimicrobial silane-containing layer disposed over the antimicrobial copper suboxide layer.
17. The coated article of clause 16, wherein the antimicrobial silane-containing layer comprises: a microbial inhibition material comprising:a first silane compound; and a metal ion; wherein the first silane compound comprises a functional group bound to the metal ion.
18. The coated article of clause 17, the first silane compound is described by Formula (I): 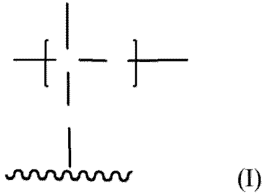 wherein: R₁ is selected from the group consisting of -C₁-Cₓ₊₁ alkyl-S(R₃)_{y}(M), -C₂-Cₓ₊₂ alkenylS(R₃)_{y}(M), -C₃-Cₓ₊₃ alkynyl-S(R₃)_{y}(M), -C₁-Cₓalkyl-N(R₃)_{y}(M), -C₂-Cₓ alkenyl-N(R³)_{y}(M), -C₃-Cₓ alkynyl-N(R₃)_{y}(M), -C₁-Cₓ₊₁ alkyl-P(R₃)_{y}(M), -C₂-Cₓ₊₂ alkenyl-P(R₃)_{y}(M), -C₃-Cₓ₊₃ alkynyl-P(R₃)_{y}(M); each R₂ is the same or different and is a bond to another Si atom of a repeating structure, or is selected from the group consisting of hydrogen, -C₁-C₁₀ alkyl, -C₂-C₁₀ alkenyl, -C₃-C₁₀ alkynyl; n is a positive integer; x is a positive integer; y is a positive integer; and M is a metal ion or metal.
19. The coated article of clause 17, wherein the antimicrobial silane-containing layer further includes a fingerprint inhibition material.
20. A coated article comprising: a substrate; an adhesion layer disposed over the substrate, the adhesion layer including a copper oxide; and an antimicrobial copper suboxide layer disposed over the adhesion layer wherein the antimicrobial copper suboxide layer is at least 5 times thicker than the adhesion layer and wherein the combination of the adhesion layer and the antimicrobial copper suboxide layer are substantially free of other metals other than copper.
21. The coated article of clause 20, wherein the antimicrobial copper suboxide layer includes a compound having formula Cu_{z}O where z is from 2.2 to 1.1.
22. The coated article of clause 20, wherein the antimicrobial copper suboxide layer includes cuprous oxide.
23. The coated article of clause 20, wherein the adhesion layer includes a compound having formula CuₓO where x is from 2.2 to 0.6.
24. The coated article of any of clauses 20 to 23, wherein a copper atom percent in the antimicrobial copper suboxide layer decrease at increasing distances from the substrate.
25. The coated article of any of clauses 20 to 24, wherein the antimicrobial copper suboxide layer has a thickness greater than 0.5 microns.
26. The coated article of any of clauses 20 to 24, wherein the antimicrobial copper suboxide layer has a thickness from about 0.5 microns to about 10 microns.
27. The coated article of any of clauses 20 to 26, wherein the antimicrobial copper suboxide layer has a gray non-iridescent color.
28. The coated article of any of clauses 20 to 27, wherein the adhesion layer has a thickness less than about 250 nm.
29. The coated article of any of clauses 20 to 27, wherein the adhesion layer has a thickness from about 10 nm to about 200 nm.
30. The coated article of any of clauses 20 to 29, wherein the antimicrobial copper suboxide layer includes a plurality of crystalline columns extending from the substrate.
31. The coated article of any of clauses 20 to 30, wherein the antimicrobial copper suboxide layer has color space coordinates L* from 35 to 55, a* from -5 to 5, and b* from -10 to -4 for illuminants D65.
32. The coated article of any of clauses 20 to 31, further comprising an antimicrobial silane-containing layer disposed over the antimicrobial copper suboxide layer.
33. The coated article of clause 32, wherein the antimicrobial silane-containing layer comprises: a microbial inhibition material comprising: a first silane compound; and a metal ion; wherein the first silane compound comprises a functional group bound to the metal ion.
34. The coated article of clause 33, the first silane compound is described by Formula (I): 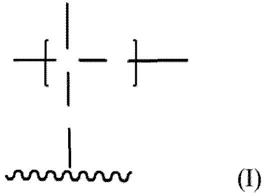 wherein: R₁ is selected from the group consisting of -C₁-Cₓ₊₁ alkyl-S(R₃)_{y}(M), -C₂-Cₓ₊₂ alkenylS(R₃)_{y}(M), -C₃-Cₓ₊₃ alkynyl-S(R₃)_{y}(M), -C₁-Cₓ alkyl-N(R₃)_{y}(M), -C₂-Cₓ alkenyl-N(R₃)_{y}(M), -C₃-Cₓ alkynyl-N(R₃)_{y}(M), -C₁-Cₓ₊₁ alkyl-P(R₃)_{y}(M), -C₂-Cₓ₊₂ alkenyl-P(R₃)_{y}(M), -C₃-Cₓ₊₃ alkynyl-P(R₃)_{y}(M); each R₂' is the same or different and is a bond to another Si atom of a repeating structure, or is selected from the group consisting of hydrogen, -C₁-C₁₀ alkyl, -C₂-C₁₀ alkenyl, -C₃-C₁₀ alkynyl; n is a positive integer; x is a positive integer; y is a positive integer; and M is a metal ion or metal.
35. The coated article of clause 33, wherein the antimicrobial silane-containing layer further includes a fingerprint inhibition material.

## Claims

1. A coated article (10) comprising:
a substrate (12);
at least one adhesion layer (14) disposed over the substrate, the at least one adhesion layer including a component selected from the group consisting of a copper oxide, a zirconium oxide, and combinations thereof; and
an antimicrobial copper suboxide layer (18) disposed over the at least one adhesion layer wherein the antimicrobial copper suboxide layer is at least 5 times thicker than the at least one adhesion layer.

2. The coated article of claim 1, wherein the antimicrobial copper suboxide layer includes a compound having formula Cu_{z}O where z is from 2.2 to 1.1.

3. The coated article of claims 1 or 2, wherein the antimicrobial copper suboxide layer includes cuprous oxide.

4. The coated article of any one of the preceding claims, wherein the at least one adhesion layer includes a compound having formula CuₓO where x is from 2.2 to 0.6, or wherein the at least one adhesion layer includes a compound having formula Zr_{y}O₂ where y is from 3 to 0.6.

5. The coated article of any one of the preceding claims, wherein the antimicrobial copper suboxide layer has a uniform stoichiometry to within 20 mole % throughout the antimicrobial copper suboxide layer.

6. The coated article of any one of the preceding claims, wherein a copper atom percent in the antimicrobial copper suboxide layer decreases at increasing distances from the substrate.

7. The coated article of any one of the preceding claims, wherein the antimicrobial copper suboxide layer has a thickness greater than 0.5 microns, optionally a thickness from about 0.5 microns to about 10 microns.

8. The coated article of any one of the preceding claims, wherein the at least one adhesion layer has a thickness less than about 250 nm, optionally from about 10 nm to about 200 nm.

9. The coated article of any one of the preceding claims, wherein the antimicrobial copper suboxide layer includes a plurality of crystalline columns extending from the substrate.

10. The coated article of any one of the preceding claims, wherein the antimicrobial copper suboxide layer has color space coordinates L* from 35 to 55, a* from -5 to 5, and b* from -10 to -4 for illuminants D65.

11. The coated article of any one of the preceding claims, further comprising a transparent metal oxide layer (20) disposed over the antimicrobial copper suboxide layer, or further comprising an antimicrobial silane-containing layer (20) disposed over the antimicrobial copper suboxide layer.

12. The coated article of claim 11, wherein the antimicrobial silane-containing layer comprises:
a microbial inhibition material comprising:
a first silane compound; and
a metal ion;
wherein the first silane compound comprises a functional group bound to the metal ion.

13. The coated article of claim 12, the first silane compound is described by Formula (I): 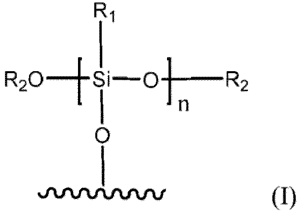 wherein:
R₁ is selected from the group consisting of -C₁-Cₓ₊₁ alkyl-S(R₃)_{y}(M), -C₂-Cₓ₊₂ alkenyl-S(R₃)_{y}(M), -C₃-Cₓ₊₃ alkynyl-S(R₃)_{y}(M), -C₁-Cₓ alkyl-N(R₃)_{y}(M), -C₂-Cₓ alkenyl-N(R³)_{y}(M), -C₃-Cₓ alkynyl-N(R₃)_{y}(M), -C₁-Cₓ₊₁ alkyl-P(R₃)_{y}(M), -C₂-Cₓ₊₂ alkenyl-P(R₃)_{y}(M), -C₃-Cₓ₊₃ alkynyl-P(R₃)_{y}(M);
each R₂ is the same or different and is a bond to another Si atom of a repeating structure, or is selected from the group consisting of hydrogen, -C₁-C₁₀ alkyl, -C₂-C₁₀ alkenyl, -C₃-C₁₀ alkynyl;
n is a positive integer;
x is a positive integer;
y is a positive integer; and
M is a metal ion or metal.

14. The coated article of claim 12, wherein the antimicrobial silane-containing layer further includes a fingerprint inhibition material.

15. A coated article (10) comprising:
a substrate (12);
an adhesion layer (14) disposed over the substrate, the adhesion layer including a copper oxide; and
an antimicrobial copper suboxide layer (18) disposed over the adhesion layer wherein the antimicrobial copper suboxide layer is at least 5 times thicker than the adhesion layer and wherein the combination of the adhesion layer and the antimicrobial copper suboxide layer are substantially free of other metals other than copper.
